(19) Europäisches Patentamt
European Patent Office
Office européen des brevets

(11) **EP 4 530 981 A1**

(12) **EUROPEAN PATENT APPLICATION**

(43) Date of publication:
**02.04.2025 Bulletin 2025/14**

(21) Application number: 23200430.9

(22) Date of filing: **28.09.2023**

(51) International Patent Classification (IPC):
*G06T 7/30* (2017.01)

(52) Cooperative Patent Classification (CPC):
**G06T 7/30;** G06T 2207/10072; G06T 2207/20016;
G06T 2207/20081; G06T 2207/30004

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Siemens Healthineers AG**
**91301 Forchheim (DE)**

(72) Inventors:
• **Ghesu, Florin-Cristian**
**91083 Baiersdorf (DE)**
• **Reaungamornrat, Sureerat**
**Havertown, 19083 (US)**
• **Wohlfahrt, Patrick**
**91054 Erlangen (DE)**

(74) Representative: **Siemens Healthineers**
**Patent Attorneys**
**Postfach 22 16 34**
**80506 München (DE)**

(54) **MULTI-ORGAN REGISTRATION SYSTEM AND METHOD**

(57) The invention relates to a system for the registration of medical imaging data with multiple organs of a patient, the system comprising: an input module which is configured to obtain imaging data of at least one tissue part of at least one organ of the patient at a time point; a computing module which is configured to compute at least one spatial mapping between the obtained imaging data and corresponding reference imaging data, wherein the at least one spatial mapping is determined based on a point distance measure and a feature-based measure, wherein the at least one spatial mapping is designed to incorporate an adaptive spatial support, and wherein the reference imaging data is obtained from a database or obtained by the input module corresponding to a different time point and/or to a different patient than the imaging data; a registration module which is configured to process the obtained imaging data and the corresponding reference imaging data using the at least one computed spatial mapping and to generate a registration information; and an output module which is configured to provide the generated registration information.

FIG 1

**EP 4 530 981 A1**

**Description**

The present invention relates to a system for the registration of imaging data related to tissue parts of multiple organs of a patient. The invention further relates to a corresponding method, a computer program product and a non-transient computer-readable data storage medium.

Herein and in the forthcoming, independent of the grammatical term usage, individuals with male, female or other gender identities are included within the term.

Registration of medical imaging data is one of the main tools in digital medicine, allowing the combination of information acquired from different imaging systems, or acquired at different time points, or acquired from different patients. By fusing information acquired from different imaging systems, comprehensive understanding of patient anatomy and/or tissue functionalities can be achieved. Alternatively, aligning images acquired at different time points allows transferring of anatomical and/or functional information between the two time points; for example, the transferring of radiotherapy planning data defined in a pre-operative computed tomography (CT) to an intra-operative cone-beam computed tomography (CBCT) immediately prior to treatment facilitates the precise localization of critical structures of interest as well as target tumours. Additionally, registering images acquired from different patients enable an atlas reconstruction, population study (e.g., variations of organs among population), and disease study (e.g., changes in anatomy due to disease).

Registration is a process in which spatial mappings between a pair of images are determined. One of the images, e.g., capturing the state/setup of the patient anatomy of interest, is often referred to as a reference image, while the other image is referred to as a moving image, often of high-quality and comprising more comprehensive anatomical/functional information.

The majority of registration algorithms estimate a single spatial mapping that brings the moving image into alignment with the reference image, thereby augmenting the reference image with more comprehensive anatomical/-functional information from the moving image, although the estimation of the mapping bringing the reference image into alignment with the moving image is feasible. Additionally, while the development of algorithms for single-organ registration or whole-image registration is rather satisfactory, the registration of multiple organs poses a number of challenges, which so far have not been resolved.

In multi-organ registration, the degrees of deformation, prominent motion directions, and the speed of deformation of parts of organs or of organs with respect to other organs can vary substantially. The varying degrees of allowed motion within one organ or the sharp changes in motion of adjacent organs make the modelling of such deformations extremely challenging. For example, the myocardium can be deformed via a shearing motion but not via a scaling motion, muscles close to a bone are more rigid than those further away from the bone, or the upper lung lobes are not as expandible as the lower lung lobes. Furthermore, organs which are close to each other can in some cases be deformed nearly independently of each other. The bladder, for example, can expand considerably when filled up with urine compared to the nearly imperceptible changes in size undergone by the surrounding structures (e.g. the uterus, the prostate or the rectum).

Prior art solutions, such as the ones that adopt B-spline functions or radial basis functions (RBFs) are either adapted to model the deformations of tissue at a local level or at a global level. In the first case, the small support of the transformation is not adapted to capture large coherent motion. In the second case, large supports cannot capture sharp motion changes. Other existing solutions, such as the ones based on non-parametric deformations or the ones based on biomechanical finite-element models (FEMs), strongly depend on image quality, deformation boundary conditions, and precise knowledge of the tissue biomechanical properties, respectively.

Against this background, the problem addressed by the present invention is to provide a system for the registration of multiple organs capable of modelling the complex motion of organs and their interactions.

This problem is solved according to the invention by a system having the features of claim 1 and/or by a computer-implemented method having the features of claim 13 and/or by a computer program product having the features of claim 14 and/or by a non-transient computer-readable data storage medium having the features of claim 15.

A first aspect of the invention provides a system for the registration of medical imaging data with multiple organs of a patient, the system comprising: an input module which is configured to obtain imaging data of at least one tissue part of at least one organ of the patient at a time point; a computing module which is configured to compute at least one spatial mapping between the obtained imaging data (D1) and corresponding reference imaging data, wherein the at least one spatial mapping is determined based on a point distance measure and a feature-based measure, wherein the at least one spatial mapping is designed to incorporate an adaptive spatial support, and wherein the reference imaging data is obtained from a database or obtained by the input module (10) corresponding to a different time point and/or to a different patient than the imaging data; a registration module which is configured to process the obtained imaging data and the corresponding reference imaging data using the at least one computed spatial mapping and to generate a registration information; and an output module which is configured to provide the generated registration information.

Imaging data broadly describes any information that can be used for further processing and analysis in medical applications of digital medicine. In the context of the present invention, the imaging data may consist of one or more scanned images of soft tissue and/or bone samples of organs of a mammal, e.g., computed tomography (CT) images or magnetic resonance (MR) images. Some of these images may comprise a selected sub-image portion or a crop of a larger image, where segmentation or tessellation has been applied. Imaging data can also comprise metadata, e.g., in the form of annotations.

Image registration is a process through which one or two spatial transformations between two images are estimated, allowing a combination of anatomical/functional information captured by the two images via, e.g., image overlay and/or image fusion. The majority of registration algorithms seek a single spatial mapping associating the pixels of a moving image (the obtained imaging data, typically having high image quality with more comprehensive anatomical/-functional information) to the pixels of a reference image (typically having lower quality but capturing the state/setup of patient anatomy of interest). The spatial mapping, in this case, therefore maps pixels, collections of pixels or regions of the moving-image domain to corresponding pixels, collections of pixels or regions of the reference-image domain. The present invention, however, allows estimation of (i) a mapping from a moving image to a reference image, or (ii) a mapping from the reference image to the moving image, or (iii) both the mapping from the moving image to the reference image and the mapping from the reference image to the moving image.

A point distance measure and a feature-based measure (or dissimilarity measure) refers to a mathematically expressible comparison of the distance between different points or of the features of different points. A point here refers commonly to a pixel or a collection of pixels. The measure can also be referred to as a metric or a distance.

The spatial support of a function is the spatial extension where the function is nonzero. An adaptive spatial support refers to the possibility of varying the spatial support of a function, typically through one or more control parameters.

The registration generated by the output module can be in the form of a graphical realization of the aligned or registered moving-image domain and/or the aligned or registered reference-image domain, if the mapping from the reference-image domain to the moving-image domain is also estimated.

The different modules and units mentioned in this application are broadly understood as entities capable of acquiring, obtaining, receiving or retrieving generic data and/or instructions through a user interface and/or programming code and/or executable programs or any combination thereof. In particular, the computing module is adapted to run programming code and executable programs and to deliver the results for further processing.

The different modules and units mentioned hereafter, or parts thereof, may therefore each contain, at least, a central processing unit, CPU, and/or at least one graphics processing unit, GPU, and/or at least one field-programmable gate array, FPGA, and/or at least one application-specific integrated circuit, ASIC and/or any combination of the foregoing. Each of them may further comprise a working memory operatively connected to the at least one CPU and/or a non-transitory memory operatively connected to the at least one CPU and/or the working memory. Each of them may comprise or consist of an application program application (API).

All the parts of the system of the invention may be realized in hardware and/or software, cable-bound and/or wireless, and in any combination thereof. Any of the parts of the system may comprise an interface to an intranet or the Internet, to a cloud computing service, to a remote server and/or the like.

In particular, each of the modules and units of the system, or the system as a whole, may be implemented partially and/or completely in a local apparatus, e.g. a computer, in a system of computers and/or partially and/or completely in a remote system, in particular in an edge or cloud computing platform.

In systems based on cloud computing technology, a large number of devices is connected to a cloud computing system via the Internet. The devices may be located in a remote facility connected to the cloud computing system. For example, the devices can comprise, or consist of, equipment, sensors, actuators, robots, and/or machinery in an industrial set-up(s). The devices can be medical devices and equipment in a healthcare unit. The devices can be home appliances or office appliances in a residential/commercial establishment.

The cloud computing system may enable remote configuring, monitoring, controlling, and maintaining connected devices (also commonly known as 'assets'). Also, the cloud computing system may facilitate storing large amounts of data periodically gathered from the devices, analyzing the large amounts of data, and providing insights (e.g., Key Performance Indicators, Outliers) and alerts to operators, field engineers or owners of the devices via a graphical user interface (e.g., of web applications). The insights and alerts may enable controlling and maintaining the devices, leading to efficient and fail-safe operation of the devices. The cloud computing system may also enable modifying parameters associated with the devices and issues control commands via the graphical user interface based on the insights and alerts.

The cloud computing system may comprise a plurality of servers or processors (also known as 'cloud infrastructure'), which are geographically distributed and connected to each other via a network. A dedicated platform (hereinafter referred to as 'cloud computing platform') is installed on the servers/processors for providing above functionality as a service (hereinafter referred to as 'cloud service'). The cloud computing platform may comprise a plurality of software programs executed on one or more servers or processors of the cloud computing system to enable delivery of the requested service to the devices and its users.

One or more application programming interfaces (APIs) are deployed in the cloud computing system to deliver various cloud services to the users.

A second aspect of the present invention provides a computer-implemented method for the registration of medical imaging data with multiple organs of a patient, comprising the following steps: obtaining imaging data of at least one tissue part of at least one organ of the patient at a time point; computing at least one spatial mapping between the obtained imaging data and corresponding reference imaging data, wherein the at least one spatial mapping is determined based on a point distance measure and a feature-based measure, wherein the at least one spatial mapping is designed to incorporate an adaptive spatial support, and wherein the reference imaging data is obtained from a database or obtained by the input module corresponding to a different time point and/or to a different patient than the imaging data; generating a registration information by processing the obtained imaging data and the corresponding reference imaging data using the at least one computed spatial mapping; and providing the generated registration information.

In particular, the method according to the second aspect of the invention may be carried out by the system according to the first aspect of the invention. The features and advantages disclosed herein in connection with the computing device are therefore also disclosed for the method, and vice versa.

According to a third aspect, the invention provides a computer program product comprising executable program code configured to, when executed, perform the method according to the second aspect of the present invention.

According to a fourth aspect, the invention provides a non-transient computer-readable data storage medium comprising executable program code configured to, when executed, perform the method according to the second aspect of the present invention.

The non-transient computer-readable data storage medium may comprise, or consist of, any type of computer memory, in particular semiconductor memory such as a solid-state memory. The data storage medium may also comprise, or consist of, a hard disk drive, a CD, a DVD, a Blu-Ray-Disc, an USB memory stick or the like.

According to a fifth aspect, the invention provides a data stream comprising, or configured to generate, executable program code configured to, when executed, perform the method according to the second aspect of the present invention.

One of the main ideas underlying the present invention is to provide a system able to resolve the deformation of multiple organs recorded with medical imaging data. The solution provided in the present invention incorporates the combination of a point distance measure and an image feature-based measure (or dissimilarity measure) in at least one spatial mapping, whose support can be adapted to the complexity of the organ movements and interactions to be registered. A spatial mapping can map the reference imaging data to the moving imaging data, or the moving imaging data to the reference imaging. These spatial mapping are not exclusive, i.e., the invention can process both at the same time.

The system as described above allows for a simple implementation of a computer-implemented method for the registration of medical imaging data with multiple organs of a patient. In one step, imaging data containing multiple organs of a patient is obtained at a certain time point. This imaging data can be generated, e.g., by a CT device. In another step, the imaging data is compared to corresponding reference imaging data and at least one spatial mapping is computed. The reference imaging data can be generated by the same CT device at a different time point or generated by a different imaging system. The reference imaging data can be acquired from a different patient. This spatial mapping is determined from a mathematical function that combines a distance between pixels and the dissimilarity of the images based on generic features, such as the image intensity or a modality-invariant image descriptor. Additionally, the spatial mapping is designed to adapt to different supports, such that a sequence of different spatial mappings for different support regions of the multiple organs captured in the imaging data can be generated. In a subsequent step, registration information (or a registration) of the obtained imaging data is generated based on a processing of the obtained imaging data using at least one computed spatial mapping. In another step, the generated registration information is provided.

One advantage of the present invention is that it provides an adaptable or even self-adaptable tool to capture sharp motion changes and large coherent motion in adjacent organs, which makes it possible to perform registration of medical imaging data for multiple organs. The system and method of the invention are modality-independent and can be applied to generic organs and tissues with different physical properties.

A further advantage of the present invention is that it incorporates different mechanisms that enable to reduce the computational demand. One of them is a sampling function that selects a number of representative points for the inference of the spatial mapping(s). Another one is the incorporation of multi-support functions that afford calculations with sparse and block matrices, which speed up the computations.

Advantageous embodiments and further developments follow from the dependent claims as well as from the description of the different preferred embodiments illustrated in the accompanying figures.

According to some embodiments, refinements, or variants of embodiments, the point distance measure comprises a comparison of the distance between points of the imaging data and corresponding points of the reference imaging data, preferably a comparison based on a continuous representation of point clouds (such as anisotropic Gaussian mixtures), and/or the feature-based measure comprises a comparison of image features between points of the imaging data and corresponding points of the reference imaging data.

A point cloud is a discrete set of data points that capture the geometry of three-dimensional structures. Prior work combined functions with scalable supports using landmark- and/or mesh-based registration, where the point correspondence is known. The combination of multi-support functions with mixture-based registration in which a discrete point set is represented by an anisotropic Gaussian mixture according to the present invention extends the capabilities of multi-support functions. Using mixture-based registration to correlate the different points enables a calculation of spatial mappings where large number of noisy partially observed surface points with unknown correspondence and unequally numbers of points between sets are involved. This increases the applicability and accuracy with which the spatial mapping can be computed.

According to some embodiments, refinements, or variants of embodiments, the at least one spatial mapping is determined by the minimization of a weighted sum of the point distance measure and the feature-based measure, wherein the weights are predetermined or treated as variables.

The determination of the spatial mapping can take place through the minimization of a function. In preferred embodiments of the invention, this function can be expressed mathematically as

$$L = \gamma_d [d(\psi'(X_0), X_1) + d(X_0, \psi(X_1))] + \gamma_s [S(\mathcal{D}(I_0 \circ \psi), \mathcal{D}(I_1)) + S(\mathcal{D}(I_0), \mathcal{D}(I_1 \circ \psi'))]$$

where $X_0 \subseteq \Omega_0$ and $X_1 \subseteq \Omega_1$ denote point clouds in the moving-image and reference-image domains. The spatial mapping function can be parametrized as

$$\psi(x_{1,k}) = t + A x_{1,k} + \sum_{i=1}^{N} w_i U \left( \|p_i - x_{1,k}\|_2 \right)$$

in terms of the affine coefficients $A$ and $\vec{t}$, the warp coefficients $W = \{\vec{w}_i\}_{i=1}^{N}$ associated with $N$ control (or sample) points $P = \{\vec{p}_i\}_{i=1}^{N}$, and a function $U(\|p_i - x_{1,k}\|_2)$ with adaptive support.

The function L contains a mapping $\psi(X_1)$ from the reference-image domain to the moving-image domain and the (inverse) mapping $\psi'(X_0)$ from the moving-image domain to the reference-image domain. The computing module finds the values of the parameters of the functions $\psi$ and $\psi'$ that minimize the function L.

The point distance measure is denoted by $d(\cdot, \cdot)$, which in some preferred embodiments can be based on an LP distance between the mixture representations of the two point sets $X_0$ and $X_1$ after transformation, as mentioned above. Besides a fast anisotropic Gaussian mixture distance between point clouds, any other point distance measure that does not assume point correspondence and an equal number of points in each point set, such as the so-called Chamfer and Hausdorff distances, can be used.

The feature-based measure contains a dissimilarity operator $S(\cdot, \cdot)$, whose argument is an image function $\mathcal{D}(\cdot)$, which can be a scalar function or a multimodality image feature function (a vector-valued function). An example of a simple scalar image feature measure can be the image intensity, in which case $\mathcal{D}(\cdot)$ is the identity operator, $\mathcal{D} = Id$. Examples of multimodality image-feature operators are the Modality Independent Neighbourhood Descriptor (MIND) used in M. Heinrich, et al, Medical Image Analysis, 16(7):1423-1435, 2012 or the Self-Similarity Context (SSC) used in M. Heinrich, et al., Medical Image Computing and Computer-Assisted Intervention, pp 187-194, 2013. The dissimilarity operator $S(\cdot, \cdot)$ can be measured in those cases using an $L^p$ distance or a robust Huber distance, for example. If the image quality is high and the registration is done between images of the same imaging modality, image intensity itself with typically an $L^p$ or a Huber loss can be used. Other image features often used in image registration can similarly be adopted.

In medical imaging, point clouds are typically derived from the segmentation of organs/structures, which may not always be available for all structures in an image, especially for fine structures, e.g., for a detailed soft tissue contrast of kidneys captured in a magnetic resonance scan or for the detailed identification of vessel structures in contrast-enhanced CT of the liver. Using image features provides a way to compensate for the lack of point cloud information in certain instances. However, image intensity has its limitations and does not always faithfully represent anatomical structures (i.e., it might be confounded by noise, artifacts, low resolution, etc.). For this reason, the weights $\gamma_d$ and $\gamma_s$ control the respective strengths of point and image dissimilarity terms in the function $L$.

According to some embodiments, refinements, or variants of embodiments, the computing module comprises a machine-learning unit, configured to implement a model of unsupervised learning, preferably a deep learning model or more preferably a deep reinforcement learning model.

Herein and in the forthcoming, a machine learning unit is to be understood as a computerized entity able to implement different data analysis methods broadly described under the terms artificial intelligence, machine learning, deep learning or computer learning. The machine learning unit can comprise an artificial neural network (or encoder). An artificial neural network is every computing system based on a collection of connected units or nodes aggregated into layers able to transmit signals to each other. The artificial neural network can be a multilayer perceptron network (MLP), a recurrent neural network (RNN), a long short-term memory (LSTM) network, a convolutional neural network (CNN), a denoising diffusion probabilistic model (DDPM) or any other neural network, including those that are based, at least partially, on a transformer neural network architecture.

According to some embodiments, refinements, or variants of embodiments, the system of the invention further comprises a scaling module, configured to provide a registration strategy based on a number of registration levels, where each registration level is characterized by a spatial support.

The adaptive spatial support that the spatial mapping incorporates can be further leveraged by defining a set of registration levels, where each registration level uses a different spatial support. The scaling module can therefore define, based on the characteristics and complexity of the imaging data to be registered, a sequence of registration levels that optimize the multi-organ registration. The scaling module can be manually or automatically operated. In these embodiments, a user can specify the different registration levels to be performed. In some other preferred embodiments, the registration strategy can be determined with the help of an algorithm, which can be aided, e.g., by the machine learning unit of the computing module. In these embodiments, the sequence of spatial supports can be defined as a hyperparameter to be determined through a deep learning or a deep reinforcement learning model. Additionally or alternatively, the support of the registration levels can be taken as one of the variables of the spatial mapping to be computed.

An example of a registration strategy can be as follows: a first registration level with large support is used as a coarse registration level, followed by registration levels with smaller support for finer registration. A sequence of registration levels with decreasing support permits the modeling of the different extent of the deformation experienced by different organs (e.g., smooth and nearly-global motion of spine and local motion bladder due to urine fill-up) as well as the modelling of abrupt changes of motion of adjacent structures (e.g., motion of lung lobes relative to motion of ribs).

According to some embodiments, refinements, or variants of embodiments, the system of the invention further comprises a sampling module, configured to select a set of sample points, which are used to evaluate the point distance measure and/or the feature-based measure.

In order to optimize the computational demand of the computing module, the dissimilarity image function and/or the point distance measure can be evaluated on a set of prominent points. Point prominence could be judged using shape (e.g., curvature) and/or texture information derived from image intensity and/or (distance transformation of) image segmentation and/or their derivatives. For instance, a sampling function could be defined based on the first and second derivatives of the distance transformation. Sample points can be defined as those whose first derivatives are zero or as those whose second derivatives having all positive, all negative, or nonzero (both positive and negative) eigenvalues for local minima, local maxima, or saddle points, respectively.

According to some embodiments, refinements, or variants of embodiments, the system of the invention further comprises a refinement module, configured to select at least one sub-region of the obtained imaging data for the one or more spatial mappings to be computed.

To further improve the alignment in challenging areas, the invention provides, in some preferred embodiments, a sub-region registration refinement. Sub-regions in the moving-image domain $\{U_n\} \subset \Omega_0$ and in the reference-image domain $\{V_m\} \subset \Omega_1$ could be defined, e.g., as bounding boxes or unions of organ segmentations. The refinement involves the determination of the parameters of $\psi$ and/or $\psi'$ that minimize the function $L$ on sample points from the interior of the corresponding sub-region.

According to some embodiments, refinements, or variants of embodiments, the refinement module is configured to define a sub-region by the requirement that there is no deformation at its boundaries.

The spatial mappings within sub-regions for a sub-region registration refinement can be defined as deformations estimated under the additional constraint $\partial V_m \circ \psi = \partial V_m$ and $\partial U_n \circ \psi' = \partial U_n$, i.e., imposing that there is no deformation at the region boundaries. This definition of a deformation-invariant boundary constraint ensures a physically plausible motion coherence between the inside and the outside points of the sub-regions. Mathematically, the constraint implies, for $\psi$, that at the boundary of the sub-region, $A = I$, $t = 0$ and $w = 0$ (and the same for $\psi'$). This hard constraint on the boundaries thus reads $\psi(x_{1,j}) = x_{1,j}$ and $\psi(x_{0,j}) = x_{0,j}$.

In some embodiments of the invention, a soft constraint can be imposed instead of a hard one, in order to allow for more flexibility at the boundaries. This soft constraint can be defined as

$$\mathcal{P}_\partial = \sum_{x_{1,j} \in \mathsf{U}_m \partial V_m} \left\| \psi(x_{1,j}) - x_{1,j} \right\|_{L^1} + \sum_{x_{0,k} \in \mathsf{U}_n \partial U_n} \left\| \psi'(x_{0,k}) - x_{0,k} \right\|_{L^1}$$

where $U_m \partial V_m$ is the union of $\{\partial V_m\} \in \Omega_1$ and $U_n \partial U_n$ is the union of $\{\partial U_n\} \in \Omega_0$.

According to some embodiments, refinements, or variants of embodiments, the spatial support is controlled by a scale parameter, which is set by a user as a hyperparameter or considered a variable. The scale parameter is a scalar value, which is introduced in the spatial mapping and it generates a family of spatial mapping functions depending on the value of the spatial support. One can either fix its value or infer it at test time as part of the minimization of the function *L* described above.

According to some embodiments, refinements, or variants of embodiments, the spatial mappings are chosen from a set of multi-support compactly supported radial basis functions (CSRBFs). RBF are especially efficient when it comes to interpolate non-uniformly scattered points. In the literature, one often finds the Wendland, Wu, Bahmann or Gneiting forms, which are given, respectively, by

$$U(r; \alpha) = \left(1 - (r/\alpha)\right)_+^4 (4(r/\alpha) + 1)$$

$$U(r; \alpha) = \left(1 - (r/\alpha)\right)_+^5 (5(r/\alpha)^4 + 25(r/\alpha)^3 + 48(r/\alpha)^2 + 40(r/\alpha) + 8)$$

$$U(r; \alpha) = 2(r/\alpha)^4 \log(r/\alpha) - 7/2(r/\alpha)^4 + 16/3(r/\alpha)^3 - 2(r/\alpha) + 16$$

$$U(r; \alpha) = \left(1 - (r/\alpha)\right)_+^4 (1 + 4(r/\alpha) - 15(r/\alpha)^2)$$

where the parameter $\alpha$ is an example of a scale parameter that controls the adaptive spatial support. The spatial mapping therefore takes the form

$$\psi\left(x_{1,k}\right) = t + Ax_{1,k} + \sum_{i=1}^{N} w_i U\left(\left\|p_i - x_{1,k}\right\|_2; \alpha\right)$$

where $U(\|p_i - x_{1,k}\|_2; \alpha)$ can be given by any CSRBF, including the examples cited above.

According to some embodiments, refinements, or variants of embodiments, the spatial mapping is determined by the use of geometric priors and/or transformation constraints and/or tissue properties, which include at least a topology-preserving constraint.

In addition to the features already noted, the determination of the spatial mapping(s) can also be improved in terms of geometric accuracy if some priors are provided. These priors can comprise a landmark-matching constraint $\mathcal{P}_L$, a tissue-dependent motion via a sliding-interface constraint $\mathcal{P}_S$, an incompressibility constraint $\mathcal{P}_I$, a topology-preserving constraint $\mathcal{P}_T$ (e.g., an invertibility constraint), and a rigid-body motion constraint $\mathcal{P}_R$ (which can in particular take into account orthogonality, properness, and/or affinity constraints, $\mathcal{P}_R = w_o \mathcal{P}_o + w_p \mathcal{P}_p + w_a \mathcal{P}_a$). These geometric priors can be each weighted and added as

$$R = w_L \mathcal{P}_L + w_S \mathcal{P}_S + w_I \mathcal{P}_I + w_T \mathcal{P}_T + w_R \mathcal{P}_R$$

The function to be minimized can then be modified to take into account these priors as

$$E = \underset{\psi, \psi'}{\text{argmin}} \, \lambda_L L + R$$

If a soft boundary constraint is imposed, it can be implemented by adding a term $w_\partial \mathcal{P}_\partial$ to the prior function *R*.

According to some embodiments, refinements, or variants of embodiments, the system of the invention is configured to be implemented into a globally-supported registration system, wherein the system of the invention is adapted to provide the globally supported registration system with adaptive spatial support. The features of the invention can be added to existing approaches that deploy B-spline functions or RBF functions (which are not providing, on their own, adaptive spatial support), thereby improving their ability to resolve challenging multi-organ motion. In these embodiments, the computer program product according to the third aspect of the invention can be provided as an Add-On to these globally-supported registration systems.

Although here, in the foregoing and also in the following, some functions are described as being performed by modules or units, it shall be understood that this does not necessarily mean that such modules or units are provided as entities separate from one another. In cases where one or more modules or units are provided as software, the modules or units may be implemented by program code sections or program code snippets, which may be distinct from one another but which may also be interwoven or integrated into one another.

Similarly, in cases where one or more modules or units are provided as hardware, the functions of one or more modules or units may be provided by one and the same hardware component, or the functions of several modules or units may be distributed over several hardware components, which need not necessarily correspond to the modules or units. Thus, any apparatus, system, method and so on which exhibits all of the features and functions ascribed to a specific module or unit shall be understood to comprise, or implement, said module or said unit. In particular, it is a possibility that all modules or units are implemented by program code executed by the computing device, for example a server or a cloud computing platform.

Where appropriate, the above-mentioned configurations and developments can be combined implementations can be combined with each other as desired, as far as this is reasonable.

Further possible configurations, developments and implementations of the invention also include combinations, which are not explicitly mentioned, of features of the invention which have been described previously or are described in the following with reference to the embodiments. In particular, in this case, a person skilled in the art will also add individual aspects as improvements or supplements to the basic form of the present invention.

The present invention is described in greater detail in the following on the basis of the embodiments shown in the schematic figures of the drawings, in which:

Fig. 1      is a schematic depiction of a multi-organ registration system for the registration of medical imaging data with multiple organs of a patient according to an embodiment of the present invention;

Fig. 2      is a block diagram showing an exemplary embodiment of a computer-implemented method for the registration of medical imaging data with multiple organs of a patient according to an embodiment of the present invention;

Fig. 3      is a schematic example of point sampling for calculating an image feature dissimilarity measure, where the delineation of structures of interest is not available;

Fig. 4      is a schematic example of a registration strategy of the thorax of a patient using the adaptive spatial support according to some embodiments of the present invention;

Fig. 5      is a depiction of multi-organ motion estimated by the computing module of the present invention, demonstrating the ability of the spatial mappings in capturing the varying degree of deformation experienced by adjacent organs in the thorax and the abdomen as well as modelling highly localized deformation and nearly-independent large translations of different organs in body cavities;

Fig. 6      is a schematic comparison of the tissue-dependent deformation obtained with a conventional registration system and with an embodiment of the system according to the present invention;

Fig. 7      is a schematic depiction of a globally-supported registration system and the system according to the first aspect of the invention, wherein the system is implemented in the globally-supported registration system and adapted to provide it with adaptive spatial support;

Fig. 8      is a schematic block diagram illustrating a computer program product according to an embodiment of the third aspect of the present invention; and

Fig. 9      is a schematic block diagram illustrating a nontransitory computer-readable data storage medium according to an embodiment of the fourth aspect of the present invention.

The appended drawings are intended to provide further understanding of the embodiments of the invention. They illustrate embodiments and, in conjunction with the description, help to explain principles and concepts of the invention. Other embodiments and many of the advantages mentioned become apparent in view of the drawings. The elements in the drawings are not necessarily shown to scale.

In the drawings, like, functionally equivalent and identically operating elements, features and components are provided with like reference signs in each case, unless stated otherwise.

The numeration of the steps in the methods are meant to ease their description. They do not necessarily imply a certain ordering of the steps. In particular, several steps may be performed concurrently.

The detailed description includes specific details for the purpose of providing a thorough understanding of the present invention. However, it will be apparent to those skilled in the art that the present invention may be practised without these specific details.

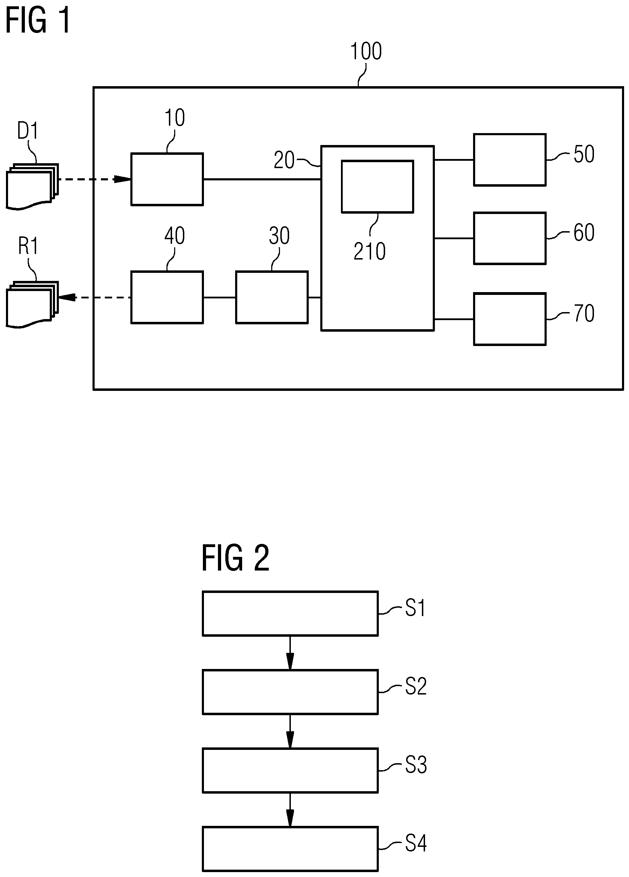

Fig. 1 shows a schematic depiction of a multi-organ registration system 100 for the registration of medical imaging data with multiple organs of a patient according to an embodiment of the present invention.

The system 100 depicted in Fig. 1 comprises an input module 10, a computing module 20, a registration module 30, an output module 40, a scaling module 50, a sampling module 60 and a refinement module 70.

The input module 10 is configured to obtain moving imaging data D1 and reference data of at least one tissue part of at least one organ of the patient, generated at different time points, or generated using a different imaging system. In some embodiments of the invention, the reference data can be of a different patient. The imaging data D1 and the reference imaging data can stem from a magnetic resonance scan or a computed tomography scan, e.g., of the thorax of a patient.

The computing module 20 is configured to compute one or more spatial mappings between the obtained imaging data D1 and corresponding reference imaging data.

The spatial mapping can be determined through the minimization of a function like

$$ L = \gamma_d \big[ d(\psi'(X_0), X_1) + d(X_0, \psi(X_1)) \big] + \gamma_s \big[ S\big(\mathcal{D}(I_0 \circ \psi), \mathcal{D}(I_1)\big) + S\big(\mathcal{D}(I_0), \mathcal{D}(I_1 \circ \psi')\big) \big] $$

which, according to the invention, comprises a point distance measure and a feature-based measure. Transformation priors can also be incorporated. The point distance measure comprises a comparison of the distance between points of the imaging data D1 and corresponding points of the reference imaging data, preferably a comparison based on a point cloud representation. The feature-based measure comprises in turn a comparison of image features between points of the imaging data D1 and corresponding points of the reference imaging data.

The spatial mapping additionally incorporates adaptive spatial support, through which the spatial extension where the function is applicable can be varied.

The computation of the computing module 20 can be based on one or more algorithms. For the computation of the point distance measure, a number of control (or sample) points can be selected. Likewise, the computation of the feature-based measure can be done with a number of sample points, chosen according to some criteria. In some preferred embodiments, the function to be minimized comprises a weighted sum of the point distance measure and the feature-based measure, where the weighting factors can be determined as part of the minimization procedure.

The spatial support of the spatial mappings can be controlled with a number of parameters. In some preferred embodiments of the invention, each spatial mapping is parametrized with compactly supported radial basis functions (CSRBFs), adapted in such a way that the spatial support can be gauged with a single scalar parameter. This parameter can be predetermined or fixed by any of the used algorithms implemented in the computing module 20.

In some embodiments of the invention, such as the one depicted in Fig. 1, the computing module 20 comprises a machine-learning unit 210, configured to implement a model of unsupervised learning, preferably a deep learning model or more preferably a deep reinforcement learning model.

The registration module 30 is configured to process the imaging data D1 and the reference imaging data obtained by the input module 10 using the spatial mapping(s) computed by the computing module 20, and therewith generate a registration information R1.

The output module 40 is configured to provide the generated registration information R1. The output module 40 can comprise a user interface, where the generated registration information R1 can be visualized by a user.

In the embodiment depicted in Fig. 1, the system 100 of the invention further comprises a scaling module 50, a sampling module 60, and a refinement module 70.

The scaling module 50 is configured to provide a registration strategy based on a number of registration levels, where each registration level is characterized by a spatial support. The sampling module 50 can thus design a sequence of spatial supports for an optimal registration. This sequence can be communicated to the computing module 20, such that one or more spatial mappings are generated for each registration level.

The sampling module 60 is configured to select a set of sample points, which are used to evaluate the point distance measure and/or the feature-based measure. With this selection of points the computational demand of the computing module 20 can be optimized. The selection of prominent points can be done following different criteria. One such possible criterion could be to define a sampling function based on the first and/or second derivatives of the distance transformation. Sample points can be defined as those whose first derivatives are zero or whose second derivatives have all positive, all negative, or nonzero eigenvalues (having both positive and negative values) representing local minima, local maxima or saddle points, respectively, of the structures of interest. This choice of sample points can be communicated to the computing module 20, in order to compute the point-distance measure and/or the feature-based measure of the spatial mappings.

The refinement module 70 is configured to select at least one sub-region of the obtained imaging data D1 for the spatial mappings to be computed. With the refinement module 70 the alignment can be improved, especially in challenging areas of the imaging data, where the motion of the different organs has to be carefully characterized (e.g., motion exhibiting abrupt change in magnitude or direction such as the motion of lung lobes relative to ribs). A sub-region can be defined by the requirement that there is no deformation at its boundaries. The choice of sub-regions can be communicated to the computing module 20, such that one or more spatial mappings are generated for each sub-region, i.e. the parameters of the functions $\psi$ and $\psi'$ are determined that minimize the function L on sample points from the interior of the corresponding sub-region.

Fig. 2 is a block diagram showing an exemplary embodiment of a computer-implemented method for the registration of medical imaging data with multiple organs of a patient according to an embodiment of the present invention. The method can be preferably implemented with the multi-organ registration system 100 described with respect to Fig. 1. The method comprises a number of steps.

In one step S1, imaging data D1 of at least one tissue part of at least one organ of a patient at a time point is obtained. The imaging data D1 can be acquired by a medical scan imaging device, such as a computed tomography (CT) device or a magnetic resonance imaging (MRI) device. Reference imaging data, which can be taken at a different time points or taken using a different imaging system, or acquired from a different patient, can also be obtained.

In another step S2, one or two spatial mappings (i.e., $\psi$, or $\psi'$ or both $\psi$ and $\psi'$) between the moving imaging data and corresponding reference imaging data D1 are computed. These spatial mappings can be determined based on the minimization of a function which comprises a point distance measure, a feature-based measure under transformation constraints, which in some preferred embodiments enter the function as a weighted sum. The spatial mappings $\psi$ and $\psi'$ are additionally adapted to be able to cover different supports, such that step S2 can be repeated for the same imaging data, taking into account the different adjustments of the spatial support of the spatial mappings. In some embodiments of the invention, a sequence of such registration levels is determined by a scaling module 50. This registration strategy can be implemented or supported by machine learning methods.

In a step S3, a registration information R1 of the obtained moving imaging data D1 and reference imaging data is generated. This registration information R1 is based on processing the moving imaging data D1 and the reference imaging data with the computed spatial mapping or mappings.

In a step S4, the generated registration information R1 is provided. The registration information R1 can be adapted to be visualized by a user, for instance through a user interface.

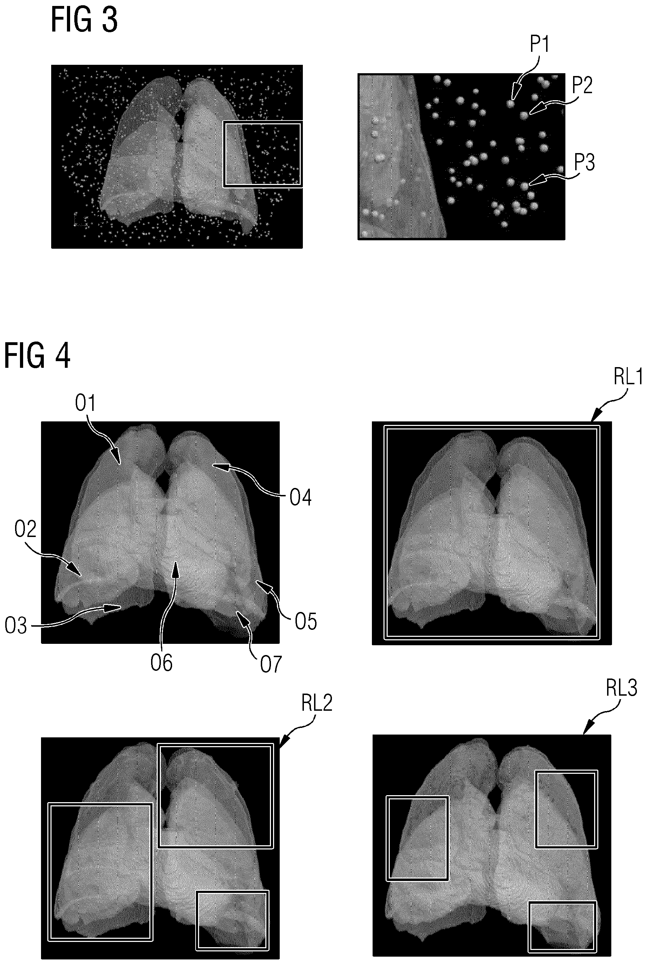

Fig. 3 shows a schematic example of sample points P1 to P3 in areas where a point cloud registration is compromised and information about, e.g., the image intensity can be used for the registration. A number of such exemplary sample points P1 to P3, used to compute the feature-based measure of the spatial mappings, are shown. The right panel of Fig. 3 is a blown-up view of the squared region in the left panel of Fig. 3.

Fig. 4 shows a schematic example of a registration strategy of the thorax of a patient using the adaptive spatial support according to some embodiments of the present invention.

The image in the upper left of Fig. 4 shows the overlay of the moving imaging data D1 and the reference imaging data of the thorax of a patient obtained with a CT device, where the moving image shows lung lobes at a time point of complete inhalation and the reference image shows lung lobes at a time point of full exhalation. In the image are shown the right superior lung lobe O1, the right middle lung lobe O2, the right inferior lung lobe O3, the left superior lung lobe O4, the left inferior lung lobe O5, the heart O6, and the spleen O7 of the patient. The reference imaging data and the moving imaging data D1 are taken from the same patient but at different times. In the moving imaging data D1, the lung lobes O1-O5 are much smaller than in the reference imaging data. The heart O6 and the spleen O7, in contrast, have barely changed their size, but, due to the breathing motion of the patient, large nearly independent translations of the heart O6 and the spleen O7 are observable from the difference in their spatial locations in the moving imaging data D1 and the reference imaging data.

The images on the upper right, lower left and lower right of Fig. 4 show a possible registration strategy according to the principles of the invention, in order to provide a registration of the imaging data D1 and the reference imaging data taking into account the presence of the multiple organs O1 to O7. This registration strategy can be generated with the scaling module 50 of the invention.

The image on the upper right of Fig. 4 shows a first registration level RL1 with large support. This could correspond, for instance, to the scale parameter $\alpha$, which controls the spatial support of the spatial mappings, taking the value $\alpha = 1$ (for the image spatial domains whose coordinates in all $x$-,$y$-, and $z$- directions are normalized to the range [-1,1]).

This first registration level RL1 would correspond to a coarse registration level, aimed at capturing mostly the nearly-global coherent motion of the organs O1 to O7. The corresponding spatial mappings are computed by the computing module 20 of the present invention.

The image on the lower left of Fig. 4 corresponds to a second, finer registration level RL2, where $\alpha < 1$. At this level, due to the smaller support of the spatial mappings, registration focuses on aligning individual structures of interest as noticeable from improved alignment of each structure of interest O1 to O7. Additionally, thanks to the smaller supports (and a sliding-interface constraint), the spatial mapping can better capture different local expansions experienced by each lung lobe as well as nearly-independent translation experienced by the heart O6 and the spleen O7.

The image on the lower right of Fig. 4 corresponds to a third registration level RL3, where $\alpha \ll 1$. At this level, thanks to the fine support of the spatial mappings, local misalignment and motion of detailed structures (including structures not represented by point clouds but included in calculating the image feature dissimilarity measure) are corrected. The use of this registration level RL3, additionally, steers transformations to better capture independent motion of organs (e.g., the motion of the lung lobes O1-O5 relative to the heart O6 and the spleen O7) and abrupt changes in the magnitude and direction of the motion (e.g., large motion of inferior lung lobes relative to superior lung lobes). With this third registration level RL3, the relative motion of the all organs O1 to O7 can be specifically resolved.

For every registration level RLi (RL1 to RL3), the computing module 20 computes corresponding spatial mappings $\psi_i$ and/or $\psi_i'$ that minimize the function $L$. The values of the parameters of the spatial mappings $\psi_i$ and $\psi_i'$ will be different for the different registration levels RL1 to RL3, since they correspond to different values of the scale parameter $\alpha$. The CSRBF functions used in the parameterization of the spatial mappings $\psi_i$ and $\psi_i'$ for the different registration levels RL1 to RL3 do not need to be the same. The final deformations are the composition of the spatial mappings estimated at each registration level, e.g., $\psi = \psi_3 \circ \psi_2 \circ \psi_1$ (the same applies for $\psi'$).

On the basis of the spatial mappings calculated for the different registration levels RL1 to RL3, the output module 30 can generate a registration information R1 of the initial imaging data D1.

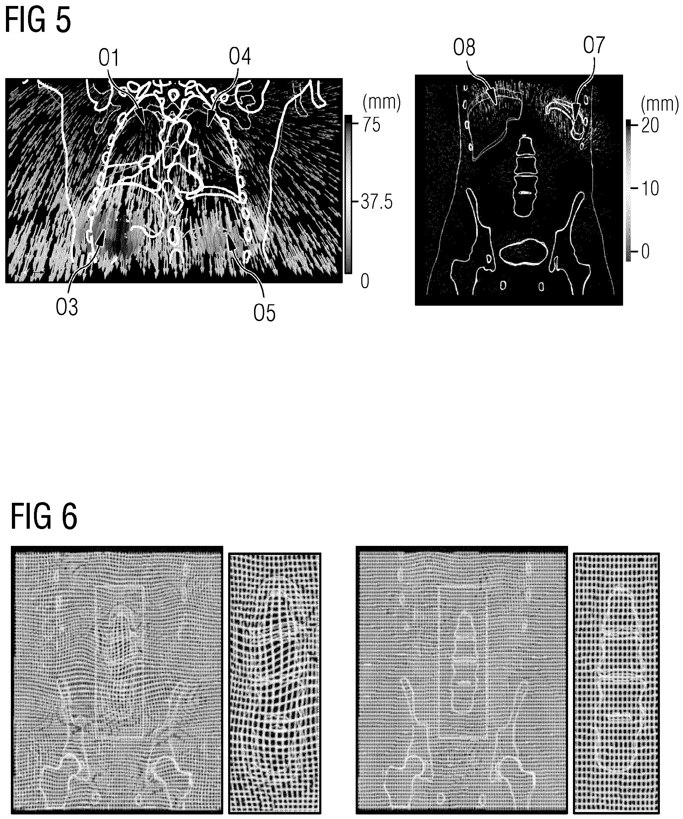

Fig. 5 depicts an example of the displacement fields (the deformation experienced from the moving-image domain compared to the reference-image domain) obtained with the system 100 according to an embodiment of the invention, capturing the complex motion of multiple organs in the thorax and the abdomen.

The left image of Fig.5 depicts motion parameterized by the multi-support spatial mapping of organs in the thoracic cavity between complete inhalation and full exhalation. The multi-support spatial mapping demonstrates the ability to model varying degrees of deformation experienced by the different lung lobes O1-O5 with the inferior lung lobes O3 and O5 exhibiting the better ability to expand and compress compared to the superior lung lobes O1 and O4, and middle lung lobe O2. Additionally, the spatial mapping can model the impact of the motion of the inferior lung lobes O3 and O5 on the nearly-independent large translations of the liver and the spleen, with the magnitude of local motion > 75 mm. The multi-support spatial mapping obtained with an embodiment of the system 100 according to the invention can, therefore, model complex independent motion of organs in the thoracic cavity.

The right image of Fig.5 depicts motion parameterized by the multi-support spatial mapping of organs in the abdominal cavity during dual energy CT imaging. Since the difference in the acquisition times of the low-energy CT and high-energy CT images is very small, the only motion observed is the motion of upper abdominal organs (e.g., the liver, the spleen, and the kidneys), due to respiratory motion, as noticeable from the misalignment of the contours of organs, such as the liver O8 and the spleen O7 in the moving imaging data and the reference image data. Thanks to multiple supports, the spatial mapping demonstrates its ability to model highly localized motion of the liver O8 and the spleen O7 within stationary surroundings as observable from the displacements having zero magnitude everywhere except at the liver and the spleen.

Fig. 6 shows a schematic comparison of the deformation (displacement field) obtained with a conventional registration system and with an embodiment of the system according to the present invention.

The leftmost image of Fig. 6 shows the deformation calculated with a conventional registration system without tissue dependent and geometric constraints. The absence of such constraints leads to failure to capture the rigidity of the vertebrae, as shown in the second image from the left, which corresponds to a blown-up view of the spine (central region) of the leftmost image.

The third image from the left of Fig. 6 shows the deformation obtained with an embodiment of the system 100 according to the invention. In contrast to the registration with a conventional registration system, the rigidity of the bone structures is successfully captured. The rightmost image of Fig. 6 shows a blown-up view of the vertebrae, whose rigidity has been preserved. This can be achieved with the principles described for the present invention, in particular with the combination of a point distance, a feature-based measure and prior knowledge regarding the tissue and deformation properties in a multi-support spatial mapping, which enables the system to have adaptive spatial support and provide different registration levels, together with the possibility of imposing geometric priors and define sub-regions for registration refinement.

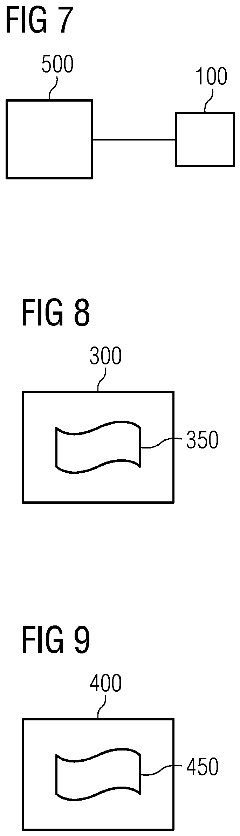

Fig. 7 shows a globally-supported registration system 500 and the system 100 according to the first aspect of the invention. The globally-supported registration system 500 can be any registration system of medical imaging data whose spatial mappings are based on B-spline functions or RBF functions which have a fixed support. The system 100 is adapted to provide the globally-supported registration system 500 with adaptive spatial support, thereby improving their ability to resolve challenging multi-organ motion. The system 100 can be coupled globally-supported registration system 500 wirelessly or connected to the globally-supported registration system 500 with a cable.

Fig. 8 shows a schematic block diagram illustrating a computer program product 300 according to an embodiment of the third aspect of the present invention. The computer program product 300 comprises executable program code 350 configured to, when executed, perform the method according to any embodiment of the second aspect of the present invention, in particular as it has been described with respect to the preceding figures.

Fig. 9 shows a schematic block diagram illustrating a non-transitory computer-readable data storage medium 400 according to an embodiment of the fourth aspect of the present invention. The data storage medium 400 comprises executable program code 450 configured to, when executed, perform the method according to any embodiment of the second aspect of the present invention, in particular as it has been described with respect to the preceding figures.

The non-transient computer-readable data storage medium may comprise, or consist of, any type of computer memory, in particular semiconductor memory such as a solid-state memory.

The data storage medium may also comprise, or consist of, a CD, a DVD, a Blu-Ray-Disc, an USB memory stick or the like.

The previous description of the disclosed embodiments are merely examples of possible implementations, which are provided to enable any person skilled in the art to make or use the present invention. Various variations and modifications of these embodiments will be readily apparent to those skilled in the art, and the generic principles defined herein may be applied to other embodiments without departing from the spirit or scope of the present disclosure. Thus, the present invention is not intended to be limited to the embodiments shown herein but it is to be accorded the widest scope consistent with the principles and novel features disclosed herein. Therefore, the present invention is not to be limited except in accordance with the following claims.

**Claims**

1. A system (100) for the registration of medical imaging data with multiple organs (O1 to O8) of a patient, the system (100) comprising:

    an input module (10) which is configured to obtain imaging data (D1) of at least one tissue part of at least one organ of the patient at a time point;
    a computing module (20) which is configured to compute at least one spatial mapping between the obtained imaging data (D1) and corresponding reference imaging data, wherein the at least one spatial mapping is determined based on a point distance measure and a feature-based measure, wherein the at least one spatial mapping is designed to incorporate an adaptive spatial support, and wherein the reference imaging data is obtained from a database or obtained by the input module (10) corresponding to a different time point and/or to a different patient than the imaging data (D1);
    a registration module (30) which is configured to process the obtained imaging data (D1) and the corresponding reference imaging data using the at least one computed spatial mapping and to generate a registration information (R1);
    and an output module (40) which is configured to provide the generated registration information (R1).

2. The system according to claim 1, wherein the point distance measure comprises a comparison of the distance between points of the imaging data (D1) and corresponding points of the reference imaging data, preferably a comparison based on a continuous representation of point clouds, and/or the feature-based measure comprises a comparison of image features between points of the imaging data (D1) and corresponding points of the reference imaging data.

3. The system according to any of the previous claims, wherein the spatial mapping is determined by the minimization of a weighted sum of the point distance measure and the feature-based measure, wherein the weights are predetermined or treated as variables.

4. The system according to any of the previous claims, wherein the computing module (20) comprises a machine-learning unit (210), configured to implement a model of unsupervised learning, preferably a deep learning model or more preferably a deep reinforcement learning model.

5. The system according to any of the previous claims, further comprising a scaling module (50), configured to provide a registration strategy based on a number of registration levels (RL1, RL2, RL3), wherein each registration level (RL1, RL2, RL3) is **characterized by** a spatial support.

6. The system according to any of the preceding claims, further comprising a sampling module (60), configured to select a set of sample points (P1, P2, P3), which are used to evaluate the point distance measure and/or the feature-based measure.

7. The system according to any of the previous claims, further comprising a refinement module (70), configured to select at least one sub-region of the obtained imaging data (D1) for the spatial mapping to be computed.

8. The system according to claim 7, wherein the refinement module (70) is configured to define a sub-region by the requirement that there is no deformation at its boundaries.

9. The system according to any of the previous claims, wherein the spatial support is controlled by a scale parameter, which is set by a user as a hyperparameter or considered a variable.

10. The system according to any of the previous claims, wherein the spatial mappings are chosen from a set of multi-support compactly supported radial basis functions.

11. The system according to any of the previous claims, wherein the spatial mapping is determined by the use of geometric priors and/or transformation constraints and/or tissue properties, which include at least a topology-preserving constraint.

12. The system according to any of the previous claims, configured to be implemented into a globally-supported registration system (500), wherein the system (100) is adapted to provide the globally-supported registration system (500) with adaptive spatial support.

13. A computer-implemented method for the registration of medical imaging data with multiple organs (O1 to O8) of a patient, preferably to be implemented with the system (100) according to any of the claims 1 to 12, comprising the steps:

   obtaining (S1) imaging data (D1) of at least one tissue part of at least one organ of the patient at a time point; computing (S2) at least one spatial mapping between the obtained imaging data (D1) and corresponding reference imaging data, wherein the at least one spatial mapping is determined based on a point distance measure and a feature-based measure, wherein the at least one spatial mapping is designed to incorporate an adaptive spatial support, and wherein the reference imaging data is obtained from a database or obtained by the input module (10) corresponding to a different time point and/or to a different patient than the imaging data (D1); generating (S3) a registration information (R1) by processing the obtained imaging data (D1) and the corresponding reference imaging data using the at least one computed spatial mapping; and providing (S4) the generated registration information (R1).

14. A computer program product (300) comprising executable program code (350), which is configured, when executed, to perform the computer-implemented method according to claim 13.

15. A non-transient computer-readable data storage medium (400) comprising executable program code (450), which is configured, when executed, to perform the computer-implemented method according to claim 13.

FIG 1

FIG 2

# FIG 3

# FIG 4

# FIG 5

# FIG 6

FIG 7

500

100

FIG 8

300

350

FIG 9

400

450

Europäisches
Patentamt

European
Patent Office

Office européen
des brevets

# EUROPEAN SEARCH REPORT

**Application Number**

EP 23 20 0430

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 111 091 589 A (BEIJING INSTITUTE TECH) 1 May 2020 (2020-05-01) | 1-3,5-7, 9,11-15 | INV. G06T7/30 |
| Y | * section "Technical field" * | 4,10 | |
| A | * page 2 * * equations (3)-(7) * * figure 2 * | 8 | |
| X | HE YUANBO ET AL: "Hierarchical anatomical structure-aware based thoracic CT images registration", COMPUTERS IN BIOLOGY AND MEDICINE, NEW YORK, NY, US, vol. 148, 14 July 2022 (2022-07-14), XP087161874, ISSN: 0010-4825, DOI: 10.1016/J.COMPBIOMED.2022.105876 [retrieved on 2022-07-14] * abstract * * sections 1, 2 * * figures 1, 2 * | 1-3,5-7, 9,11-15 | |
| X | LOC PHAM XUAN ET AL: "Multi-resolution Coarse-to-fine Registration Approach for Liver Computed Tomography Image Analysis", 2022 26TH INTERNATIONAL COMPUTER SCIENCE AND ENGINEERING CONFERENCE (ICSEC), IEEE, 21 December 2022 (2022-12-21), pages 307-312, XP034301048, DOI: 10.1109/ICSEC56337.2022.10049333 [retrieved on 2023-02-24] | 1,13-15 | **TECHNICAL FIELDS SEARCHED (IPC)** G06T |
| Y | * abstract * * section II * * figure 1 * | 4 | |

-/--

The present search report has been drawn up for all claims

| Place of search | Date of completion of the search | Examiner |
|---|---|---|
| Munich | 15 March 2024 | Engels, Angela |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

Europäisches Patentamt

European Patent Office

Office européen des brevets

# EUROPEAN SEARCH REPORT

**Application Number**

**EP 23 20 0430**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHU WENTAO ET AL: "NeurReg: Neural Registration and Its Application to Image Segmentation", 2020 IEEE WINTER CONFERENCE ON APPLICATIONS OF COMPUTER VISION (WACV), IEEE, 1 March 2020 (2020-03-01), pages 3606-3615, XP033771155, DOI: 10.1109/WACV45572.2020.9093506 [retrieved on 2020-05-13] * abstract * * section 3 * | 1,13-15 | |
| A | WANG YI ET AL: "Towards Personalized Statistical Deformable Model and Hybrid Point Matching for Robust MR-TRUS Registration", IEEE TRANSACTIONS ON MEDICAL IMAGING, IEEE, USA, vol. 35, no. 2, 1 February 2016 (2016-02-01), pages 589-604, XP011597615, ISSN: 0278-0062, DOI: 10.1109/TMI.2015.2485299 [retrieved on 2016-02-01] * abstract * * sections II, III * * figure 3 * | 1,13-15 | **TECHNICAL FIELDS SEARCHED (IPC)** |

-/--

The present search report has been drawn up for all claims

| Place of search | Date of completion of the search | Examiner |
|---|---|---|
| Munich | 15 March 2024 | Engels, Angela |

Europäisches
Patentamt

European
Patent Office

Office européen
des brevets

# EUROPEAN SEARCH REPORT

**Application Number**

**EP 23 20 0430**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHEN XINRONG ET AL: "ROBUST SURFACE-MATCHING REGISTRATION BASED ON THE STRUCTURE INFORMATION FOR IMAGE-GUIDED NEUROSURGERY SYSTEM", JOURNAL OF MECHANICS IN MEDICINE AND BIOLOGY, vol. 21, no. 05, 17 April 2021 (2021-04-17), page 2140009, XP093141767, ISSN: 0219-5194, DOI: 10.1142/S0219519421400091 * abstract * | 1,13-15 | |
| Y | FORNEFETT M ET AL: "Elastic registration of medical images using radial basis functions with compact support", PROCEEDINGS OF THE 1999 IEEE COMPUTER SOCIETY CONFERENCE ON COMPUTER VISION AND PATTERN RECOGNITION, JUNE 23-25, 1999; FORT COLLINS, COLORADO, IEEE, THE INSTITUTE OF ELECTRICAL AND ELECTRONICS ENGINEERS, INC, US, vol. 1, 23 June 1999 (1999-06-23), pages 402-409, XP010347689, ISBN: 978-0-7695-0149-9 * abstract * * sections 2.3 and 3 * | 10 | |

TECHNICAL FIELDS
SEARCHED (IPC)

The present search report has been drawn up for all claims

| Place of search | Date of completion of the search | Examiner |
|---|---|---|
| Munich | 15 March 2024 | Engels, Angela |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons
...........................................................................
& : member of the same patent family, corresponding
 document

EPO FORM 1503 03.82 (P04C01)

## ANNEX TO THE EUROPEAN SEARCH REPORT
## ON EUROPEAN PATENT APPLICATION NO.

**EP 23 20 0430**

This annex lists the patent family members relating to the patent documents cited in the above-mentioned European search report.
The members are as contained in the European Patent Office EDP file on
The European Patent Office is in no way liable for these particulars which are merely given for the purpose of information.

**15-03-2024**

| Patent document cited in search report | Publication date | Patent family member(s) | Publication date |
|---|---|---|---|
| CN 111091589 A | 01-05-2020 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **M. HEINRICH et al.** *Medical Image Analysis*, 2012, vol. 16 (7), 1423-1435

- **M. HEINRICH et al.** *Medical Image Computing and Computer-Assisted Intervention*, 2013, 187-194